# NOUVEAU FASCICULE DE BREVET EUROPEEN

(11) **EP 2 197 287 B2**
(45) Date de publication et mention de la décision concernant l'opposition: **12.03.2025**
(45) Mention de la délivrance du brevet: 08.01.2020
(21) Numéro de dépôt: 08858623.5
(22) Date de dépôt: 02.10.2008
(51) Int. Cl.: A23C 9/12, A23C 21/02, A23L 33/135, A61K 35/20, A61K 35/74, A23C 9/123, A23L 33/00, A61K 35/745, A61P 37/00, A61P 37/08, A61K 35/744

(54) **UTILISATION D'UNE SOUCHE DE BIFIDOBACTERIUM, POUR LA PREPARATION D'UNE COMPOSITION DESTINEE A LA PREVENTION ET/OU AU TRAITEMENT DE MANIFESTATIONS DE TYPE ALLERGIQUE**
VERWENDUNG EINES BIFIDOBAKTERIENSTAMMES ZUR HERSTELLUNG EINER ZUSAMMENSETZUNG ZUR VERHINDERUNG UND/ODER BEHANDLUNG ALLERGISCHER LEIDEN
USE OF A BIFIDOBACTERIUM STRAIN FOR PREPARING A COMPOSITION FOR PREVENTING AND/OR TREATING ALLERGIC MANIFESTATIONS

(30) Priorité: 03.10.2007 FR 0706925
(43) Date de publication de la demande: 23.06.2010
(73) Titulaire: Compagnie Gervais Danone, 75009 Paris (FR)
(72) Inventeur: AUBERT-JACQUIN, Cécile, 2581 BB Den Haag (NL); LECROIX, Francis, F-59270 Godewaersvelde (FR); PERRIN, Emmanuel, F-59299 Boeschepe (FR); PETAY, Valérie, B-8950 Heuvelland (BE)
(74) Mandataire: Gevers & Orès
(86) Numéro de dépôt international: PCT/FR2008/001372
(87) Numéro de publication internationale: WO 2009/074754

(56) Documents cités:
- NAKAMURA Y: "Agent for preventing and/or treating allergies, comprises Bifidobacterium, or its processed substance chosen from e.g. culture, concentrate and paste of Bifidobacterium, as an active ingredient", DERWENT/WPI,, 1 January 1900 (1900-01-01), XP002466730
- SANDY PENG ET AL: "Antiallergic effect of milk fermented with lactic acid bacteria in a murine animal model", JOURNAL OF AGRICULTURAL AND FOOD CHEMISTRY, AMERICAN CHEMICAL SOCIETY. WASHINGTON, US, vol. 55, no. 13, 27 May 2007 (2007-05-27), pages 5092 - 5096, XP009103004, ISSN: 0021-8561
- "Antiallergic agent for treating and preventing allergy such as atopic dermatitis - comprises enterobacterium like bifido bacterium, lactobacillus acidophilus", WPI WORLD PATENT INF,, 1 January 1900 (1900-01-01), XP002905585
- HOARAU ET AL: "Supernatant of Bifidobacterium breve induces dendritic cell maturation, activation, and survival through a Toll-like receptor 2 pathway", JOURNAL OF ALLERGY AND CLINICAL IMMUNOLOGY, MOSBY - YEARLY BOOK, INC, US, vol. 117, no. 3, 1 March 2006 (2006-03-01), pages 696 - 702, XP005396922, ISSN: 0091-6749
- PRESCOTT S L ET AL: "Clinical effects of probiotics are associated with increased interferon-gamma responses in very young children with atopic dermatitis", CLINICAL AND EXPERIMENTAL ALLERGY, BLACKWELL SCIENTIFIC PUBLICATIONS, LONDON, GB, vol. 35, no. 12, 1 December 2005 (2005-12-01), pages 1557 - 1564, XP002430722, ISSN: 0954-7894
- MULLIE C ET AL: "Partial characterization of bifidobacterium breve C50 cell-free whey compounds inducing modifications to the intestinal microflora", JOURNAL OF DAIRY SCIENCE, AMERICAN DAIRY SCIENCE ASSOCIATION, SAVOY, IL, US, vol. 85, no. 6, 1 June 2002 (2002-06-01), pages 1383 - 1389, XP002269107, ISSN: 0022-0302
- MATSUMOTO S ET AL: "PREVENTIVE EFFECTS OF BIFIDOBACTERIUM- AND LACTOBACILLUS-FERMENTED MILK ON THE DEVELOPMENT OF INFLAMMATORY BOWEL DISEASE IN SENESCENCE-ACCELERATED MOUSE P1/YIT STRAIN MICE", DIGESTION, BASEL, vol. 64, no. 2, 1 January 2001 (2001-01-01), pages 92 - 99, XP009013024, ISSN: 0012-2823
- PRIOULT G ET AL: "Allergenicity of acidic peptides from bovine beta-lactoglobulin is reduced by hydrolysis with Bifidobacterium lactis NCC362 enzymes", INTERNATIONAL DAIRY JOURNAL, ELSEVIER APPLIED SCIENCE, BARKING, GB, vol. 15, no. 5, 1 May 2005 (2005-05-01), pages 439 - 448, XP004820391, ISSN: 0958-6946
- MENARD S ET AL: "LACTIC ACID BACTERIA SECRETE METABOLITES RETAINING ANTI-INFLAMMATORY PROPERTIES AFTER INTESTINAL TRANSPORT", GUT, BRITISH MEDICAL ASSOCIATION, LONDON, vol. 53, no. 6, 1 June 2004 (2004-06-01), pages 821 - 828, XP008046639, ISSN: 0017-5749
- KIRJAVAINEN P V ET AL: "Aberrant composition of gut microbiota of allergic infants: A target of bifidobacterial therapy at weaning?", GUT, BRITISH MEDICAL ASSOCIATION, LONDON, vol. 51, no. 1, 1 July 2002 (2002-07-01), pages 51 - 55, XP009096010, ISSN: 0017-5749

## Description

La présente invention est relative à un procédé de préparation de compositions, notamment d'une formule infantile, permettant de réduire les manifestations de type allergique aux protéines de lait de vache. Ces compositions sont obtenues à partir d'une culture de *Bifidobacterium,* sans hydrolyse des protéines de lait.

Depuis plusieurs décennies, l'incidence des maladies allergiques est en augmentation dans les pays industrialisés. Ainsi, on estime aujourd'hui que 20% de la population des pays développés est touchée par une forme d'allergie comme l'asthme, la dermatite atopique, ou d'autres formes de manifestations. Cette forte incidence positionne aujourd'hui l'allergie comme un problème de santé publique et dès lors, de nombreux travaux de recherche ont été entrepris pour tenter d'apporter des solutions permettant de limiter le développement de ces pathologies. Ces travaux ont permis d'identifier les 2 premières années de vie, comme une période critique dans l'installation de la maladie, et notamment par des phases de sensibilisation aux allergènes, les enfants développant une sensibilisation pendant cette période présentant plus de risques de développer une allergie ultérieurement. Par ailleurs, le rôle de la flore intestinale dans la genèse de l'allergie commence également à être révélé. En effet, l'établissement de la flore intestinale chez l'enfant conditionne le développement du système immunitaire mucosal (Prescott et al., 2005), et les schémas de mise en place de la flore pourraient influencer l'orientation immunitaire vers de la tolérance orale ou au contraire de la sensibilisation (Kalliomaki et al., 2001; Sudo et al., 1997).

L'allergie se définit plus précisément comme une réaction d'hypersensibilité, impliquant un mécanisme immunitaire (Johansson et al., 2001). Cette hypersensibilité cause des symptômes reproductibles, déclenchés par l'exposition à un antigène, à une dose habituellement tolérée chez des sujets normaux. Les antigènes entraînant une réaction allergique, les allergènes, sont de nature très diverse, alimentaire, aériens, animaux *etc.* De même, les symptômes observés sont très variables, principalement de type cutané, digestif ou respiratoire. Les stratégies proposées en prévention de l'allergie ont pour objectif de diminuer l'incidence de nouveaux cas d'allergie (prévention primaire), ou de limiter la durée et l'évolution de l'allergie quand elle est installée (prévention secondaire).

Chez le nourrisson, les allergies les plus fréquentes sont les allergies alimentaires, et notamment, l'allergie aux protéines de lait de vache (APLV). Le risque est d'autant plus important chez les sujets atopiques (enfants à risque allergique élevé, notamment par le fait d'une hérédité allergique avérée), ce qui justifie d'une stratégie, chez ces enfants, d'intervention diététique durant la petite enfance. L'allaitement maternel exclusif prolongé (> 6 mois), associé à une diversification alimentaire progressive, est la solution de référence, l'allaitement ayant un effet potentiellement protecteur vis-à-vis de l'apparition de maladie allergique (Host and Halken, 2005). Pour les enfants à risque ne bénéficiant pas d'un allaitement maternel, une autre stratégie impliquant une dénaturation des protéines du lait de vache par hydrolyse partielle ou poussée, ou leur remplacement par d'autres protéines animales ou végétales est recommandée (Host et al., 1999). Pour autant, l'efficacité préventive de ces solutions demeure controversée (Osborn and Sinn, 2006a; Osborn and Sinn, 2006b), et le processus d'hydrolyse a pour conséquence la dégradation de l'organoleptie et de la qualité nutritionnelle des protéines, occasionnant des refus chez certains enfants. En France, les formules hydrolysées sont de plus soumises à une réglementation particulière limitant leur distribution au réseau pharmaceutique exclusivement, ce qui est une contrainte supplémentaire pour les parents de ces enfants à risque.

L'effet anti-allergénique de laits fermentés a été décrit, sous un angle mécanistique, par Peng et al. Les produits étudiés sont des laits fermentés probiotiques acidifiés (pH= 3,7) de type "yoghourt". Dans le modèle présenté, l'effet repose sur la double composante du produit testé : fermentation et maintien des ferments en vie (> 10⁹ cfu/ml). Cinq types de ferments différents ont été testés (3 *Lactobacilles*, 1 *Streptoccocus thermophilus* MC et 1 *Bifidobacterium longum*) et les auteurs ont observé des effets très variables et dépendants de la souche utilisée. Sur ce modèle animal, les effets les plus remarquables ont été observés pour les souches de lactobacilles testées. Par ailleurs, les effets observés sont de type purement immunologique, et non pas directement reliés à des observations cliniques, sur la symptomatologie de type allergique (Peng et al., 2007). A noter par ailleurs que toutes les souches probiotiques ne sont pas compatibles avec l'alimentation infantile, l'acide D lactique étant non métabolisable par les nourrissons.

Terpend *et al.* ont étudié plus spécifiquement les propriétés d'un lait fermenté par *Bifidobacterium breve* et *Streptococcus thermophilus.* Les résultats de cette étude montrent un effet de renforcement de la barrière intestinale aux protéines de lait. Toutefois, ces auteurs ne sont pas parvenus à mettre en évidence un effet anti-allergénique direct d'un tel lait fermenté (Terpend et al., 1999).

Dans ce contexte, les inventeurs ont développé des formulations adaptées à l'alimentation infantile, qui remédient à au moins une partie des inconvénients des produits décrits dans l'art antérieur, ce qui permet de proposer une approche différente dans la prise en charge diététique des enfants à risque allergique.

L'approche choisie pour apporter un bénéfice, consiste à réguler directement la mise en place de la flore et du système immunitaire intestinal de l'enfant, par l'intermédiaire de composés actifs immunomodulateurs issus de la bioconversion ou de la fermentation par une souche de *Bifidobacterium.* Les résultats, présentés dans la partie expérimentale ci-après, démontrent des bénéfices directs sur la symptomatologie allergique.

La présente invention porte donc, sur une composition pour une utilisation selon les revendications. La présente invention porte sur une composition obtenue par un procédé comprenant une étape de bioconversion d'un substrat laitier à l'aide d'une culture d'une souche de *Bifidobacterium breve*, par maintien dudit substrat en contact avec ladite culture, dans des conditions défavorables à la production d'acide par ladite souche de *Bifidobacterium*, pour une utilisation dans la prévention de manifestations de type allergique chez l'enfant ladite composition étant pour une utilisation dans la prévention d'une allergie aux protéines de lait de vache. Une souche particulièrement adaptée à la mise en oeuvre de la présente invention est la souche BbC50, déposée le 31 mai 1999 par la Compagnie Gervais Danone, auprès de la Collection Nationale de Cultures de Microorganismes (CNCM), sous le numéro I-2219.

Un procédé de préparation d'une composition destinée à la prévention et/ou au traitement de manifestations allergiques conforme à l'invention comprend, de manière avantageuse, une étape de fermentation d'un substrat laitier en présence de la souche de *Bifidobacterium.* Cette étape de fermentation d'un substrat laitier en présence de la souche de *Bifidobacterium* mentionnée ci-dessus peut être effectuée en conditions aérobies ou anaérobies.

Par "substrat laitier", on entend ici un milieu aqueux comprenant au moins une fraction de protéines de lactosérum et du lactose. Le cas échéant, les protéines de lactosérum peuvent être hydrolysées et/ou enrichies en lactalbumine native. Du perméat de lactosérum peut également être intégré dans le substrat laitier. A titre d'exemples non limitatifs de substrats laitiers, on peut citer du lait, un concentré de lait, une base pour aliment lacté infantile, une base pour yoghourt, *etc* ...

Dans une mise en œuvre préférée de l'invention, le procédé de préparation d'une composition destinée à la prévention et/ou au traitement de manifestations allergiques conforme à l'invention comprend une étape de bioconversion d'un substrat laitier par la souche de *Bifidobacterium,* réalisée dans des conditions défavorables à la production d'acide par ladite souche, comme décrit dans la demande WO 2001/001785. Cette bioconversion, qui correspond à une fermentation non acidifiante ou peu acidifiante, permet également la production de métabolites actifs. Des précisions sur les conditions de cette fermentation particulière sont indiquées ci-après.

On définit par "conditions défavorables à la production d'acide par *Bifidobacterium"* des conditions dans lesquelles l'acidification du milieu par *Bifidobacterium* n'excède pas 0,5 unités pH en 8 heures d'incubation pour un ensemencement initial supérieur à 1 x 10⁷ UFC par ml. Elles peuvent aisément être déterminées par l'homme de l'art à l'aide de simples essais, en faisant varier notamment l'aération du milieu de culture, sa pression osmotique et/ou la température de culture, et en mesurant le pH en début et en fin de culture.

Pour un grand nombre de souches de *Bifidobacterium,* de telles conditions peuvent notamment être obtenues par :
- l'utilisation d'une population bactérienne issue d'un inoculum à un stade postérieur à la phase exponentielle de croissance ;
- le maintien en conditions aérobies, par exemple sous agitation ;
- le maintien du milieu à une pression osmotique correspondant à 0,93 à 0,97 d'activité de l'eau (AW) ;
- le maintien à une température de 40 à 48°C ;
ainsi que des combinaisons de ces différentes conditions.

Dans cette mise en œuvre de l'invention, la mise en contact du substrat laitier et des bifidobactéries peut être effectuée à raison de 10⁷ à 10⁹ UFC par ml de substrat laitier, et la population finale de bifidobactéries à l'issue de la réaction de bioconversion peut être comprise entre 10⁵ et 10⁹ UFC par ml de produit.

Le pH du substrat laitier lors de la mise en contact avec les bactéries est de préférence de 6,0 à 7 et le pH du produit à l'issue de la réaction de bioconversion est préférentiellement de 5,8 à 7.

Selon les conditions utilisées, le temps de contact entre le substrat laitier et les bactéries pour une telle étape de bioconversion peut être compris entre 4 et 24 heures.

Bien entendu, dans un procédé conforme à l'invention, les étapes de bioconversion et de fermentation décrites ci-dessus ne sont pas exclusives l'une de l'autre. Par exemple, un tel procédé peut comprendre une étape de bioconversion (peu ou pas acidifiante) d'un substrat laitier par *Bifidobacterium,* suivie d'une étape de fermentation acidifiante par cette même bactérie. Alternativement, les deux réactions peuvent être menées en parallèle, sur deux substrats laitiers distincts, les produits des deux réactions étant ensuite réunis dans la composition anti-allergique finale.

Selon la nature de la composition anti-allergique que l'on souhaite obtenir, différents traitements additionnels peuvent être appliqués au produit de la bioconversion et/ou de la fermentation du substrat laitier par les bifidobactéries. En particulier, une étape de stérilisation et/ou de séparation des bifidobactéries peut être avantageusement envisagée à l'issue de l'étape de fermentation ou de bioconversion, notamment pour obtenir des produits de longue conservation. De même, différentes étapes de filtration, déshydratation ou de chromatographie peuvent être réalisées, suivant la nature de la composition finale que l'on souhaite obtenir.

Des exemples de produits utilisables pour la préparation d'une composition destinée à la prévention et/ou au traitement de manifestations allergiques, conformément à la présente invention, sont décrits notamment dans les Demandes WO 2001/001785 (produit utilisé dans l'exemple 1 ci-après), WO 2004/093898 et WO 2004/093899 (exemple 2), et WO 2006/040485. Bien entendu, des mélanges de ces produits peuvent également être utilisés conformément à l'invention.

Le cas échéant, une composition obtenue par un procédé de l'invention peut comprendre, outre le produit de la fermentation ou bioconversion d'un substrat laitier par une souche de *Bifidobacterium,* une masse résultant de la fermentation d'un substrat laitier par une souche de *Streptococcus thermophilus,* par exemple par la souche ST065, déposée le 23 août 1995 par la Compagnie Gervais Danone, auprès de la Collection Nationale de Cultures de Microorganismes (CNCM), sous le numéro I-1620. Une composition obtenue selon la présente invention peut, de manière avantageuse, contenir une fraction obtenue par un procédé tel que décrit dans la Demande WO 96/06924.

Selon une mise en œuvre préférée de l'invention, la composition obtenue est un alicament (ou aliment fonctionnel), c'est-à-dire un aliment ou ingrédient alimentaire ayant un bénéfice pour la santé, en l'occurrence au niveau de la prévention ou du traitement des manifestations allergiques. A titre d'exemples, la composition peut être un produit lacté frais de type yoghourt, un plat cuisiné, une sauce, *etc.* La composition peut également être un produit de longue conservation, stérilisé et/ou déshydraté.

Une composition obtenue selon une mise en œuvre préférée de l'invention est destinée à l'alimentation infantile. Par exemple, il peut s'agir d'un lait infantile (sous forme déshydratée ou non). Un exemple non limitatif de lait infantile obtenu conformément à la présente invention est le lait commercialisé sous le nom Gallia Calisma (1^{er} ou 2^{ème} âge) par le groupe Danone.

Il est important de noter qu'une composition obtenue selon l'un des procédés ci-dessus ou dans l'exemple 2 ci-après peut être incorporée, à titre d'ingrédient, dans n'importe quel type de composition alimentaire. Une composition alimentaire obtenue selon l'invention pourra donc être caractérisée par le fait qu'elle renferme, à titre d'ingrédient, au moins une composition obtenue selon l'un des procédés décrits ci-dessus ou dans l'exemple 2 ci-après. De telles compositions alimentaires peuvent être destinées à l'alimentation humaine ou animale et peuvent notamment se présenter sous forme d'une préparation lactée ou non, fermentée ou non, d'origine animale ou végétale, y compris notamment les formules infantiles ou pour adultes et seniors, et en particulier sous forme d'une préparation lactée infantile, de lait liquide ou en poudre, de produits frais, de céréales, de biscuits (fourrage), de petits pots, de desserts, *etc.,* ou bien encore sous forme de produits alimentaires ou diététiques pour adultes, dont les produits hospitaliers.

Alternativement, une composition obtenue selon la présente invention peut se présenter comme un complément nutritionnel.

Selon une autre mise en oeuvre, la présente invention concerne un procédé de préparation d'une composition pharmaceutique destinée à la prévention et/ou au traitement de manifestations allergiques. Une composition pharmaceutique obtenue selon la présente invention sera caractérisée par le fait qu'elle renferme, à titre de principe actif, au moins une composition obtenue selon l'un des procédés décrits plus haut ou dans l'exemple 2 ci-après, et au moins un support pharmaceutiquement acceptable. Par "pharmaceutiquement acceptable", on entend tout support qui, tout en conservant ses propriétés à la composition obtenue selon l'un des procédés décrits ci-dessus ou dans l'exemple 2 suivant, permet de véhiculer ladite composition.

Un complément nutritionnel ou une composition pharmaceutique obtenu(e) selon la présente invention peut se présenter sous toute forme galénique souhaitée pour une administration par voie orale à l'homme ou à l'animal, comme par exemple sous forme liquide pour un sirop ou une solution, un spray, ou sous forme solide comme par exemple une poudre, un comprimé, une gélule, une capsule, un spray poudre, une gomme, une pâte, des granulés, dans leurs formes diverses, à libération immédiate ou programmée, ou sous toute autre forme adaptée à l'administration par voie orale. Les compositions pharmaceutiques obtenues conformément à la présente invention peuvent en outre être formulées pour une administration par voie topique à l'homme ou à l'animal, comme par exemple sous la forme d'une crème, une lotion, un savon, une solution, un gel, un lait, une huile ou pour une administration par voie anale à l'homme ou à l'animal, comme par exemple sous la forme d'un suppositoire.

Les compositions obtenues par un procédé conforme à l'invention sont de préférence destinées à l'alimentation humaine, et apporteront un bénéfice à toutes les personnes présentant un terrain allergique. Les individus souffrant de manifestations de type allergique, ainsi que les nouveaux-nés, nourrissons ou jeunes enfants (jusqu'à 5 ans) dont au moins un des parents présente une allergie et/ou un terrain atopique, constituent une population à qui ces compositions apporteront un grand bénéfice. Les individus souffrant d'allergies alimentaires, respiratoires ou de contact, ainsi que les sujets atopiques, pourront également constater une amélioration de leur état du fait d'une consommation régulière d'une composition obtenue par un procédé conforme à la présente invention.

Les résultats de l'étude présentée dans les exemples suivants ci-après montrent en particulier que des préparations infantiles obtenues conformément à l'invention permettent d'une part, de prévenir l'apparition d'une allergie aux protéines de lait de vache (APLV) chez un certain nombre d'enfants à risque allergique élevé et, d'autre part, de traiter des manifestations cutanées de type allergique chez des nourrissons. L'utilisation d'une souche de *Bifidobacterium,* pour la préparation d'une composition destinée à la prévention de l'allergie aux protéines de lait de vache et/ou pour la préparation d'une composition destinée à la prévention et au traitement de manifestations cutanées de type allergique (eczéma, desquamation, fissure rétro-auriculaire, érythème, démangeaisons...) chez des nourrissons, fait donc partie intégrante de l'invention.

La présente invention sera mieux comprise à l'aide du complément de description qui va suivre, qui présente les effets anti-allergiques du lait infantile Gallia Calisma sur des nourrissons présentant un terrain allergique. D'autres préparations utilisables selon la présente invention sont également décrites.

### EXEMPLES

### Exemple 1 : effets anti-allergiques du lait infantile Gallia Calisma

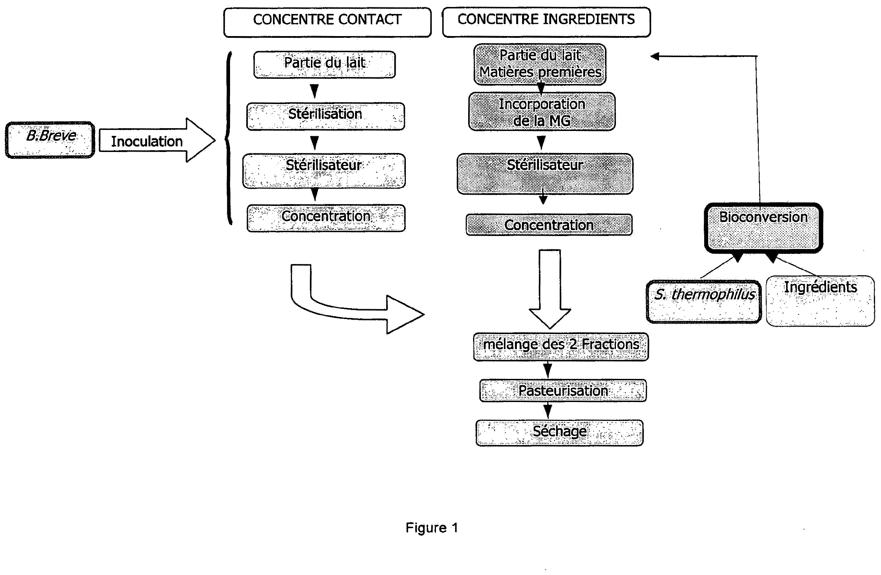

Une préparation pour nourrisson (du type Calisma 1^{er} âge) et un lait de suite (du type Calisma 2^{ème} âge) ont été intégrés dans une étude prospective, longitudinale multicentrique, randomisée en double aveugle, avec 2 groupes parallèles. Le procédé de préparation de ces compositions est schématisé à la figure 1.

L'objectif était de déterminer l'efficacité d'un lait infantile bioconverti (FF) sur la prévention de l'allergie aux protéines de lait de vache (APLV), et l'apparition de sensibilisation aux allergènes, chez le nouveau-né et le nourrisson, par rapport à une formule non bioconvertie (SF).

Des enfants à haut risque atopique ont été recrutés pour participer à cette étude. Les mères ont été recrutées avant le 5ème mois de grossesse avec un suivi de l'enfant jusqu'à 12 mois. La FF ou SF a été donnée de la naissance à 1 an ou à partir du sevrage pour les enfants qui ont été allaités. Un suivi diététique a été mené dès le 5^{ème} mois de grossesse et jusqu'aux 12 mois de l'enfant avec diversification tardive selon les recommandations actuelles. Au cours du suivi de l'enfant pendant sa première année, les manifestations compatibles avec une allergie alimentaire ont été recueillies.

Un examen clinique systématique a également été pratiqué à 4 et 12 mois ainsi que des *prick-tests* (tests cutanés mettant en évidence la sensibilité à un allergène donné) au lait de vache (LV), soja, oeuf, blé, morue, arachide, D. *pteronyssinus, Alternaria*, chat, chien, pollens de graminées et bouleau. En cas de suspicion d'APLV, tout apport de protéines de lait de vache a été interrompu et un test de provocation orale (TPO) au LV a été mené.

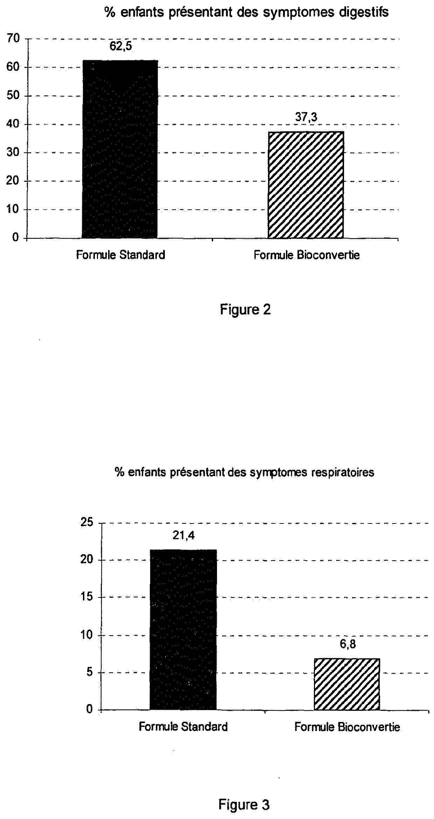

129 enfants ont été inclus, parmi lesquels 115 ont été suivis jusqu'à 12 mois, dont 59 ont consommé la FF et 56 la SF. Sur 115 patients, 83 ont présenté des symptômes compatibles avec une allergie alimentaire (72,2%).Dans le groupe FF, le pourcentage des manifestations digestives et respiratoires est observée est statistiquement plus faible que celui du groupe SF (p < 0,05). Ces résultats sont présentés dans les figures 2 et 3.

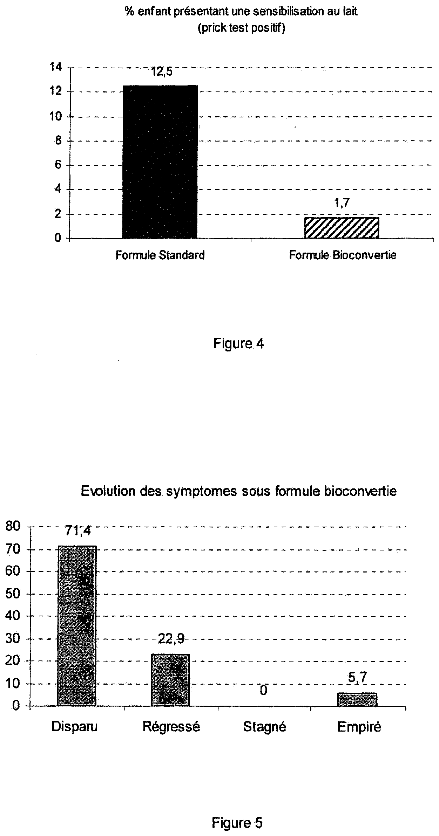

De plus, sur ces 115 enfants, 7% ont présenté une sensibilisation au lait (Prick Test positif), avec une différence significative (p<0,05) entre les groupes FF et SF (1,7% et 12,5% respectivement) (figure 4).

Cette étude a démontré pour la première fois, l'intérêt de l'utilisation d'une formule bioconvertie, en prévention de l'apparition des manifestations de l'allergie alimentaire et de la sensibilisation aux protéines de lait de vache.

La FF à également été testée au cours d'une étude prospective, longitudinale multicentrique, ouverte évaluant l'intérêt de l'utilisation d'une telle formule dans la prise en charge de la symptomatologie cutanée mineure.

Cette étude a évalué l'effet d'une formule bioconvertie spécifique (Calisma), par rapport à un lait standard en mesurant l'évolution de manifestations cutanées mineures chez des nourrissons, après remplacement du lait standard par la formule fermentée.

Des nourrissons bien portants, âgés de moins de 4 mois, présentant des manifestations cutanées de type allergique correspondant à un 1er épisode symptomatologique, ont été recrutés. Les manifestations cutanées relevées étaient les suivantes : desquamation, fissure rétro auriculaire, érythème, démangeaisons.

Après un mois de consommation de la formule bioconvertie, l'évolution de ces manifestations cutanées mineures (nature, localisation et intensité) a été évaluée par un pédiatre et par les parents.

35 enfants ont été inclus dans l'étude. Après 1 mois de consommation de la formule bioconvertie, une évolution favorable des manifestations a été observée pour 94 % des enfants, dont une disparition complète des symptômes pour 71% (figure 5).

Ces résultats montrent l'efficacité du produit dans la prise en charge de manifestations de nature allergique.

### Exemple 2 : Préparation d'autres compositions anti-allergiques

D'autres compositions anti-allergiques peuvent être obtenues par un procédé comprenant les étapes suivantes :
a- l'ensemencement et l'incubation, en conditions aérobies ou anaérobies et à une température comprise entre 30 et 40 °C environ, de *Bifidobacterium* comprenant au moins la souche *Bifidobacterium breve* I-2219 dans un substrat aqueux présentant un pH compris entre 6 et 8 environ, et comprenant au moins les ingrédients suivants :
   i) du perméat de lactosérum,
   ii) un hydrolysat de protéines de lactosérum,
   iii) du lactose
b- l'élimination des *Bifidobacterium* du substrat aqueux ;
c- l'ultrafiltration du substrat aqueux sur des membranes de filtration ayant un seuil de coupure compris entre 100 et 300 kDa pour obtenir un rétentat concentré ;
complété, le cas échéant, des étapes d- et e- ci-après :
d- la déshydratation du rétentat concentré ;
e- la mise en solution du rétentat déshydraté dans un tampon.

Enfin, les étapes f- et g- suivantes peuvent être effectuées pour obtenir des fractions actives du rétentat :
f- la chromatographie d'exclusion sur gel sur colonne présentant un seuil d'exclusion de 600 kDa de la solution du rétentat ;
g- la récupération de la fraction exclue et/ou de la fraction filtrée à l'issue de la chromatographie.

Plus précisément, les produits peuvent être obtenus en utilisant le protocole décrit ci-dessous.

On prépare un milieu de culture contenant les ingrédients suivants :
- 50 g/l de perméat de lactosérum,
- 10 g/l d'hydrolysat de protéines de lactosérum,
- 20 g/l de lactose,
- 2 g/l d'extrait de levure,
- 2,5 g/l de dihydrogénophosphate de potassium,
- 0,3 g/l de chlorhydrate de cystéine
- 0,3 g/l de NaOH.

Le milieu de culture est ultrafiltré sur cassettes Centramate^{®} vendues par la société PALL, munies de membranes en polyéthersulfone ayant un seuil de coupure de 300 kDa et le perméat est autoclavé pendant 30 minutes à 120°C. Le pH du milieu de culture est alors ajusté à une valeur de 6,5 à l'aide d'une solution d'ammoniaque diluée à la moitié.

Le milieu de culture est ensuite ensemencé avec les bifidobactéries à raison de 6 ‰ (v/v) d'un concentré congelé de la souche de *Bifidobacterium breve* CNCM I-2219 contenant 1,1.10¹¹ UFC de bifidobactéries par ml de concentré congelé. La population initiale en bactéries est de 3.10⁸ UFC de bifidobactéries par ml de milieu de culture. Les bifidobactéries sont cultivées en anaérobiose, à une température comprise entre 35 et 40°C. Pendant la culture, le pH du milieu de culture est régulé à 6,5 au moyen d'une solution d'ammoniaque diluée à la moité. Le temps de culture est de 15 heures, et la population de *Bifidobacterium* en fin de culture est environ de 2.10⁷ UFC par ml de milieu de culture.

En fin de culture, les bactéries sont éliminées du milieu de culture fermenté par une centrifugation de 1 heure à 3000 g. Les activités enzymatiques résiduelles contenues dans le surnageant de centrifugation sont détruites par un traitement thermique de 3 minutes à 75°C.

Le surnageant est ultrafiltré sur cassettes Centramate^{®} vendues par la société PALL, munies de membranes en polyéthersulfone ayant un seuil de coupure de 300 kDa à une température de 40°C environ. Il est ainsi concentré 3 fois, puis lavé 3 fois à l'eau permutée. Pendant le dernier lavage, on opère une concentration de 7 fois de la partie retenue par la membrane. On obtient ainsi un concentré appelé rétentat. Le rétentat est déshydraté par lyophilisation, puis repris dans un tampon Tris-NaCl à pH 8.

Le rétentat concentré, préalablement repris dans un tampon Tris-NaCl pH 8, est soumis à une chromatographie préparative. La séparation est réalisée par chromatographie sur colonne de gel Superdex^{®} 200 vendue par la société Amersham Biosciences de 50 mm de diamètre et 100 cm de hauteur, alimentée à un débit de 5 ml par minute et présentant un seuil d'exclusion de 600 kDa. Les fractions sont collectées par 10 ml et leur absorbance est mesurée à 280 nanomètres.

Deux fractions sont ainsi séparées :
- une fraction exclue du gel de poids moléculaire supérieur à 600 kDa (temps de rétention de 130 à 180 minutes +/- 10 %),
- une fraction filtrée de poids moléculaire compris entre 200 et 600 kDa (temps de rétention de 187 à 370 minutes +/- 10 %).

La fraction exclue est dialysée contre de l'eau distillée puis diluée de façon à revenir à la concentration du rétentat. Cette fraction peut ensuite être conservée sous forme congelée ou lyophilisée. La fraction filtrée peut également être conservée de la même manière.

Le rétentat ou l'une quelconque des fractions séparées par chromatographie, ainsi que leurs mélanges, peuvent être utilisées pour prévenir, atténuer ou traiter les manifestations de type allergique.

### REFERENCES

Host, A., and Halken, S. (2005). Primary prévention of food allergy in infants who are at risk. Curr Opin Allergy Clin Immunol 5, 255-259.
Host, A., Koletzko, B., Dreborg, S., Muraro, A., Wahn, U., Aggett, P., Bresson, J. L., Hernell, O., Lafeber, H., Michaelsen, K. F., et al. (1999). Dietary products used in infants for treatment and prévention of food allergy. Joint Statement of the European Society for Paediatric Allergology and Clinical Immunology (ESPACI) Committee on Hypoallergenic Formulas and the European Society for Paediatric Gastroenterology, Hepatology and Nutrition (ESPGHAN) Committee on Nutrition. Arch Dis Child 81, 80-84.
Johansson, S. G., Hourihane, J. O., Bousquet, J., Bruijnzeel-Koomen, C., Dreborg, S., Haahtela, T., Kowalski, M. L., Mygind, N., Ring, J., van Cauwenberge, P., et al. (2001). A revised nomenclature for allergy. An EAACI position statement from the EAACI nomenclature task force. Allergy 56, 813-824.
Kalliomaki, M., Kirjavainen, P., Eerola, E., Kero, P., Salminen, S., and Isolauri, E. (2001). Distinct patterns of neonatal gut microflora in infants in whom atopy was and was not developing. J Allergy Clin Immunol 107, 129-134.
Osborn, D. A., and Sinn, J. (2006a). Formulas containing hydrolysed protein for prévention of allergy and food intolerance in infants. Cochrane Database Syst Rev, CD003664.
Osborn, D. A., and Sinn, J. (2006b). Soy formula for prévention of allergy and food intolerance in infants. Cochrane Database Syst Rev, CD003741.
Peng, S., Lin, J. Y., and Lin, M. Y. (2007). Antiallergic effect of milk fermented with lactic acid bacteria in a murine animal model. J Agric Food Chem 55, 5092-5096.
Prescott, S. L., Dunstan, J. A., Hale, J., Breckler, L., Lehmann, H., Weston, S., and Richmond, P. (2005). Clinical effects of probiotics are associated with increased interferon-gamma responses in very young children with atopic dermatitis. Clin Exp Allergy 35, 1557-1564.
Sudo, N., Sawamura, S., Tanaka, K., Aiba, Y., Kubo, C., and Koga, Y. (1997). The requirement of intestinal bacterial flora for the development of an IgE production system fully susceptible to oral tolerance induction. J Immunol 159, 1739-1745.
Terpend, K., Blaton, M. A., Candalh, C., Wal, J. M., Pochart, P., and Heyman, M. (1999). Intestinal barrier function and cow's milk sensitization in guinea pigs fed milk or fermented milk. J Pediatr Gastroenterol Nutr 28, 191-198.

## Revendications

1. Composition obtenue par un procédé comprenant une étape de bioconversion d'un substrat laitier à l'aide d'une culture d'une souche de *Bifidobacterium breve,* par maintien dudit substrat en contact avec ladite culture, dans des conditions défavorables à la production d'acide par ladite souche de *Bifidobacterium,* pour une utilisation dans la prévention de manifestations de type allergique chez l'enfant
ladite composition étant pour une utilisation dans la prévention d'une allergie aux protéines de lait de vache.

2. Composition pour son utilisation selon la revendication 1, **caractérisée en ce que** la souche de *Bifidobacterium breve* est la souche BBC50, déposée le 31 mai 1999 auprès de la Collection Nationale de Cultures de Microorganismes (CNCM), sous le numéro I-2219.

3. Composition pour son utilisation selon l'une quelconque des revendications 1 à 2, **caractérisée en ce que** son procédé de préparation comprend en outre une étape de fermentation d'un substrat laitier en présence de ladite souche de *Bifidobacterium.*

4. Composition pour son utilisation selon l'une des revendications 1 à 3, **caractérisée en ce que** son procédé de préparation comprend une étape de stérilisation et/ou de séparation desdites *bifidobacteria* à l'issue de l'étape de fermentation ou de bioconversion.

5. Composition pour son utilisation selon l'une quelconque des revendications précédentes, **caractérisée en ce qu'**elle comprend en outre une masse résultant de la fermentation d'un substrat laitier par une souche de *Streptococcus thermophilus.*

6. Composition pour son utilisation selon la revendication 5, dans laquelle ladite souche de *Streptococcus thermophilus* est la souche ST065, déposée le 23 août 1995 auprès de la Collection Nationale de Cultures de Microorganismes (CNCM), sous le numéro 1-1620.

7. Composition pour son utilisation selon l'une quelconque des revendications précédentes, **caractérisée en ce qu'**il s'agit d'un alicament, un complément alimentaire ou une composition pharmaceutique.

8. Composition pour son utilisation selon l'une quelconque des revendications précédentes, **caractérisée en ce qu'**elle est destinée à des individus souffrant de manifestations de type allergique, à des sujets atopiques, ou à des enfants dont au moins un des parents présente une allergie et/ou un terrain atopique.

9. Composition pour son utilisation selon l'une quelconque des revendications précédentes, **caractérisée en ce qu'**elle est destinée à des nourrissons présentant des manifestations cutanées de type allergique.

## Patentansprüche

1. Zusammensetzung, erhalten durch ein Verfahren, umfassend einen Schritt der biologischen Umwandlung eines Milchsubstrats mithilfe einer Kultur eines Stamms von *Bifidobacterium breve* durch Halten des Substrats in Kontakt mit der Kultur unter Bedingungen, die für die Produktion von Säure durch den Stamm von *Bifidobacterium* ungünstig sind, für eine Verwendung bei der Prävention von Manifestationen des allergischen Typs bei Kindern
wobei die Zusammensetzung für eine Verwendung bei der Prävention einer Allergie gegen Kuhmilchproteine dient.

2. Zusammensetzung zur Verwendung nach Anspruch 1, **dadurch gekennzeichnet, dass** der Stamm von *Bifidobacterium breve* der Stamm BBC50 ist, eingereicht am 31. Mai 1999 bei der Collection Nationale de Cultures de Microorganismes (CNCM) unter der Nummer I-2219.

3. Zusammensetzung zur Verwendung nach einem der Ansprüche 1 bis 2, **dadurch gekennzeichnet, dass** ihr Zubereitungsverfahren weiter einen Fermentierungsschritt eines Milchsubstrats in Anwesenheit des Stamms von *Bifidobacterium* umfasst.

4. Zusammensetzung zur Verwendung nach einem der Ansprüche 1 bis 3, **dadurch gekennzeichnet, dass** ihr Zubereitungsverfahren einen Sterilisierungs- und/oder Abtrennungsschritt der *Bifidobakterien* nach Abschluss des Fermentierungsschritts oder der biologischen Umwandlung umfasst.

5. Zusammensetzung zur Verwendung nach einem der vorstehenden Ansprüche, **dadurch gekennzeichnet, dass** sie weiter eine Masse umfasst, die aus der Fermentierung eines Milchsubstrats durch einen Stamm von *Streptococcus thermophilus* resultiert.

6. Zusammensetzung zur Verwendung nach Anspruch 5, wobei der Stamm von *Streptococcus thermophilus* der Stamm ST065 ist, eingereicht am 23. August 1995 bei der Collection Nationale de Cultures de Microorganismes (CNCM) unter der Nummer I-1620.

7. Zusammensetzung zur Verwendung nach einem der vorstehenden Ansprüche, **dadurch gekennzeichnet, dass** es sich um ein funktionelles Lebensmittel, ein Nahrungsergänzungsmittel oder eine pharmazeutische Zusammensetzung handelt.

8. Zusammensetzung zur Verwendung nach einem der vorstehenden Ansprüche, **dadurch gekennzeichnet, dass** sie für Individuen, die unter Manifestationen des allergischen Typs leiden, für atopische Patienten oder für Kinder bestimmt ist, von denen mindestens eins der Elternteile eine Allergie und/oder eine atopische Konstitution aufweist.

9. Zusammensetzung zur Verwendung nach einem der vorstehenden Ansprüche, **dadurch gekennzeichnet, dass** sie für Kleinkinder bestimmt ist, die kutane Manifestationen des allergischen Typs aufweisen.

## Claims

1. Composition obtained by a method comprising a step of bioconverting a dairy substrate with the aid of a culture of a strain of *Bifidobacterium breve,* by maintaining said substrate in contact with said culture, under unfavourable conditions to the production of acid by said strain of *Bifidobacterium,* for use in the prevention of allergic-type manifestations in children said composition being for use in the prevention of allergy to cow's milk proteins.

2. Composition for use according to claim 1, **characterized in that** the strain of *Bifidobacterium breve* is the strain BBC50, filed on 31 May 1999 with the Collection Nationale de Cultures de Microorganismes (CNCM), under the number I-2219.

3. Composition for use according to any one of claims 1 to 2, **characterized in that** its preparation method further comprises a step of fermenting a milk substrate in the presence of said strain of *Bifidobacterium.*

4. Composition for use according to one of claims 1 to 3, **characterized in that** its preparation method comprises a step of sterilizing and/or separating said *bifidobacteria* at the end of the fermentation or bioconversion step.

5. Composition for use according to any one of the preceding claims, **characterized in that** it additionally comprises a mass resulting from the fermentation of a dairy substrate by a strain of *Streptococcus thermophilus.*

6. Composition for use according to claim 5, wherein said strain of *Streptococcus thermophilus* is the strain ST065, filed on 23 August 1995 at the Collection Nationale de Cultures de Microorganismes (CNCM), under the number I-1620.

7. Composition for use according to any one of the preceding claims, **characterized in that** it is a health food, a food supplement or a pharmaceutical composition.

8. Composition for use according to any one of the preceding claims, **characterized in that** it is intended for individuals suffering from manifestations of the allergic type, for atopic subjects or for children with at least one parent presenting an allergy and/or an atopic condition.

9. Composition for use according to any one of the preceding claims, **characterized in that** it is intended for infants with skin manifestations of the allergic type.
